# EUROPEAN PATENT SPECIFICATION

(11) **EP 3 616 687 B1**
(45) Date of publication and mention of the grant of the patent: **29.11.2023**
(21) Application number: 18729014.3
(22) Date of filing: 16.03.2018
(51) Int. Cl.: A61K 9/10, A61L 2/18, A61P 17/00, A61P 31/00, A61K 9/00, A61P 31/02, A61K 47/38, A61K 47/14, A61K 47/10, A61K 31/155, A61L 101/46, A61L 2/00

(54) **PREPARATION FOR THE TREATMENT AND/OR PROPHYLAXIS OF INFLAMMATORY INFECTIOUS DISEASES**
ZUBEREITUNG ZUR BEHANDLUNG UND/ODER PROPHYLAXE VON ENTZÜNDLICHEN INFEKTIONSERKRANKUNGEN
PRÉPARATION POUR LE TRAITER ET/OU LA PRÉVENTION DE MALADIES INFECTIEUSES ET INFLAMMATOIRES

(30) Priority: 27.04.2017 RU 2017114945
(43) Date of publication of application: 04.03.2020
(73) Proprietor: Lovtsov, Valentin Vyacheslavovich, Saratov 410017 (RU); Lukyanov, Alexander Veniaminovich, g. Moskva (RU)
(72) Inventor: LOVTSOV, Valentin Vyacheslavovich, Odintsovskiy r-n - Moskovskaya obl. 143033 (RU)
(74) Representative: Benatov, Samuil Gabriel
(86) International application number: PCT/RU2018/000163
(87) International publication number: WO 2018/106153

(56) References cited:
- CN-A- 101 332 317
- NL-A- 8 104 627
- US-A1- 2004 072 910
- Anonymous: "Zaregistrirovan v gosudarstvennom reestre lekarstvennykh sredstv, registratsionn y nomer: P N015801/01", rosminzdrav , 8 November 2009 (2009-11-08), XP055649572, Retrieved from the Internet: URL:http://grLs.rosminzdrav.ru/GrLs_View_v 2.aspx?routingGuid=1ceba65f-c113-4be5-80ca -374887487038&t= [retrieved on 2018-07-06]
- Anonymous: "Zaregistrirovan v gosudarstvennom reestre lekarstvennykh sredstv, registratsionny nomer: P N012477/01", rosminzdrav , 18 July 2008 (2008-07-18), XP055649593, Retrieved from the Internet: URL:http://grls.rosminzdrav.ru/Grls_View_v 2.aspx?routingGuid=02fed3fc-45a9-45db-ba67 -3bd1d63c8ea3&t= [retrieved on 2018-06-07]
- ANDERSEN A.: "Final amended report on the safety assessment of methylparaben, ethylparaben, propylparaben, isopropylparaben, butylparaben, isobutylparaben, and benzylparaben as used in cosmetic products", International Journal of Toxicology, vol. 27, no. 4, 2008, pages 1-82, XP009517604, DOI: 10.1080/10915810802548359

## Description

### FIELD OF INVENTION

The invention relates to the field of medicine and the chemical and pharmaceutical industry, and more particularly to antiseptic preparations

Inflammatory infectious diseases (IIDs) of the urogenital tract constitute a major part in the gynecological and andrological pathology structure, and despite of some success achieved in solving this problem, improving methods of prevention and treatment for the above diseases remains extremely relevant.

Numerous studies show that the causative agents for the genitourinary tract IIDs (vulvitis, urethritis, vaginitis, cervicitis), in the vast majority of cases, constitute a polymicrobial association, comprising both pathogenic microorganisms Chlamydia trachomatis, Neisseria gonorrhoeae, Trichomonas vaginalis, Mycoplasma genitalium, Mycoplasma hominis, and opportunistic microbial pathogens (OMP) [Dovletkhanova E.R., Abakarova P.R. / Potential use of combined preparations in the treatment of polymicrobial vulvovaginitis // Women's Health. 2013; 6: 101-2 *(in Russian)].*

High prevalence of latent and asymptomatic disease forms is a particular problem. The lack of clinical signs means that such patients do not contact health services, and, as a consequence, continuously infect their sexual partners.

Immediately after the incubation period, classical disease pattern only develops in 40-50% of those infected, with about 20% of them having paucisymptomatic infection. These factors cause late referral to healthcareprofessionals, leading inevitably to complications that affect reproductive organs.

The main causative agents of urogenital tract inflammatory diseases are found within genital tracts of most infertile couples. Infertility incidence is 50% in those with a chlamydial infection, 30% in the presence of Mycoplasma and Ureaplasma species, and about 45% in the presence of Trichomonas species. However, clinical signs of urethritis may be present in as low as 40-60% of patients [Kondratieva Y.S., Neymark A.I. / Mixed urogenital infections: clinical and therapeutic approaches / Journal of Dermatology and Venereology 2011; 4: 112-116 (in Russian*)].*

Upon sexual intercourse with a knowingly infected partner, there is a time interval between the moment of infection and that in which the infectious disease starts to develop. This time period is necessary for the microorganisms to be deposited on mucous membranes of the internal genital organs, and therefore, in prevention of sexually transmitted infections [STIs], it is important to apply the required preparation within 2 h after sexual intercourse. This "priceless" time frameis often insufficient to consult a physician and get a professional advice. However, self-administration of emergency preventive medication is possible, which would protect the "clean" partner from contamination.

The widespread prevalence of sexually transmitted diseases [STDs] necessitates the search for novel approaches to STD prevention, and the development of preparations capable of eliminating causative agents' ability to invade human body.

In this aspect, the interest has renewed, in Russian and foreign practice, in antiseptics which possess proven effectiveness and a number of advantages.

While analyzing preparations used as preventive medication against STDs, which are available on the pharmaceutical market, we noted that all of them possess both advantages and disadvantages, to a varying degree, and do not fully satisfy the demand for emergency pharmacotherapy of sexually transmitted infections. It should be noted that some of these preparations are intended predominantly for intravaginal use only [Kamaeva S.S. / Theoretical and experimental rationale for developing antimicrobial dosage forms with spermicidal activity / DSc. (Phys.) Diss. Abstr. Moscow, 2009 (in Russian*)],* while intraurethral administration of the above preparations is necessary, both in males and females, to ensure complete decontamination of mucous surfaces.

Hence, a need arises to optimize the preparation composition, form andapplication method in order to ensure maximum effect, and also for ease of use.

In our survey of drugs that can be used to prevent STD infections, we aimed at comparing preparations formulated mainly as gels, since, according to many authors, gels are incontestably superior to liquid dosage forms, i.e. solutions.

Numerous studies have proven that the action of aqueous preparations turns out to be brief, and concentration of the main active substance required to suppressthe microbial flora is often not achieved. In addition, there exist but a few preparations in the form of solution. The main representatives are Miramistine and solutions with varying Chlorhexidine content.

Experimental results confirmed significant toxicity of the Miramistine and Chlorhexidine solutions even in concentrations offered by pharmacy networks. It was shown that, despite the high efficiency of these medications against various pathogens, their long-term usage in combination treatment of inflammatory diseases may cause the repair processes not only in mucous membranes, but also in skin, to slow down. These same researches proved that gel medications are highly potent and do not have a toxic effect on cells, and therefore can be an alternative to the popular antiseptic solutions [Bogayeva V.V., Popava V.M., Pashkova G.S., et al. / Efficacy and safety studies of antimicrobial agents/ Research and Practice in Medicine. 2015, No.3, pp. 35-42 (in Russian*)].*

Trichomoniasis is one of the most common sexually transmitted infections. Effective treatment of this disease is challenging due to the shedding of atypical, inactive, anucleate form of the causative agent [Moreva Zh. G., Gerasko E. V. / Specific features of the in vitro effect of antimicrobial preparations on Trichomonas/ Bulletin of the Ivanovo Medical Academy 2006, Vol. 11, 3-4, pp 17-24 (in Russian*)].*

Notably, a number of researchers have found, relying upon experimental data, that the exposure to Miramistine antiseptic preparation makes the atypical forms of Trichomonas to go through adaptive population restructuring, which consists in the appearance of even more atypical forms, determining their high resistance. The results of the conducted research explain the lack of effectiveness why trichomoniasis prevention and treatment by using Miramistine antiseptic preparation has a suboptimal effectiveness. In addition, comparison of Miramistine and Chlorhexidine during clinical microbiological studies has proved that the Chlorhexidine antibacterial activity is 8-fold higher compared to Miramistine. The authors conclude that Chlorhexidine is currently the most promising antiseptic agent. According to a number of authors, Cathejell, Instillagel, and Hexicon are currently the main signature medications based on Chlorhexidine, which could be used as preventive medications against STDs. All of these preparations are provided in the form of gels. Hexicon preparation can be also formulated as vaginal suppositories.

Hexicon preparation consists of Chlorhexidine and excipients: Poloxamer 407, Cremophor - RH 40 (Polyoxyl 40 Hydrogenated Castor Oil), purified water [https://medi.m/instrukciya/geksikon-gel_6074/].

However, some authors note that the use of Hexicon preparation is accompanied by local irritant effect and pain syndrome. It was the itching and burning sensations, observed by researchers when using Hexicon preparation, that gave rise to discontinuation of treatment [Budanov P.V., Aslanov A.G., Musaev Z.M. / Efficient restoration of vaginal resistance to colonization: the advantage and limitation of vaginal dosage forms // Problems of Gynecology, Obstetrics and Perinatology. 2013. Vol. 12 (in Russian*)].*

In 2004, B. Molteni et al. conducted a multicenter study, wherein it was noted that 20% of the patients using Hexicon gel complained of a transient burning sensation after application thereof [Molteni B, D'Antuono A, Bandini P et al. / Efficacy and tolerability of a new Chlorhexidine-based vaginal gel in vaginal infections. // Curr. Med. Res. Opin 2004; 20 (6):849-53].

Cathejell is a topical gel comprising active substances, lidocaine hydrochloride 2 g, Chlorhexidine dihydrochloride 0.05 g, and excipients: hyetellose (hydroxyethyl cellulose) 1.5 g; glycerol 20 g; water for injections - up to 100 g (https://www.rlsnet.rU/tn_index_id_1692.htm#sostav).

Instillagel preparation comprises Chlorhexidine in combination with p-hydroxybenzoic acid ester resulting in potentiation of its properties [Hasin A.Z., Ediger A.V. Methodology aspects of Instillagel application in urological and gynecological practice. // Moscow Medical Journal 1999. No.26 (in Russian*)].* Researchers have proved that it is specifically the Instillagel antimicrobial complex (AMcI ("A 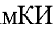 ")) that has a bactericidal action on a wide range of gram-positive and gram-negative microorganisms, as well as on trichomonads and fungi, due to the mutual potentiation of the components. This preparation may be listed as the closest analogue.

It is crucial to note that Cathejell and Instillagel combined formulations include topical anesthetic lidocaine, which, according to the creators of these preparations themselves, can lead to undesirable complications, such as bradycardia, convulsions, Quincke's edema, collapse [Basic Prescribing Information].

Furthermore, the presence oOf lidocaine translate these preparations into prescribed ones, thus making impossible their widespread use, and, accordingly, their preventive use becomes irrelevant. On the other hand, upon sexual intercourse with a knowingly diseased partner there is a time interval between the moment of infection and that in which the infectious disease starts to develop. This time periodis necessary for the microorganisms to be deposited on mucous membranes of the internal genital organs. For STI prevention, it is important to apply the required preparation within 2 h after sexual intercourse.

An antiseptic lubricant composition for use in sexual relations is known from US 2004/072910 A1. This is a water-soluble lubricant with main composition of glycerin, water, a spermicide, an antibiotic agent and a fungicide.

Hence, the disadvantages of the above preparations did not allow them tocome into wide use as emergency medications for STI prevention.

The problem in STI prevention is to develop a drug that would meet the following requirements:
- A convenient dosage form suitable for treating the mucous surfaces of the external and internal genital organs
- High antiseptic activity with no toxicity, or disturbances of genital microbial community
- Minimal side effects
- Ease of use
- No contraindications
- Over-the-counter sale

The problem is addressed by the novel inventive Chlorhexidine-based preparation.

The inventive pharmaceutical composition is distinctive not only in the method of use, but also in the qualitative and quantitative composition thereof. The novel preparation according to claim 1 is a gel comprising the following components, in grams:

| | |
|---|---|
| Chlorhexidine gluconate | 0.01-0.5 |
| methyl-4-hydroxybenzoate | 0.01-0.1 |
| ethyl-4-hydroxybenzoate | 0.01-0.1 |
| propyl-4-hydroxybenzoate | 0.01-0.1 |
| propylene glycol | 5-70 |
| hydroxyethyl cellulose | 1.5-5 |
| water | up to 100 g |

The preparation is a colorless, transparent, viscous, homogeneous gel packaged in disposable sterile syrettes with nozzles suited for the gel administrationinto a male or female urogenital tract.

Gel is one of the most convenient means of delivering a drug to a proper site. The gel is a soft dosage form and covers a mucous membrane smoothly, holds wellto it, is color and odor free, while staying on for a longer time compared to solutions.

In addition, the preparation base *per se* can qualify as a drug substance, since it softens the mucosa, has antimicrobial activity, and is able to destruct biofilms.

Chlorhexidine, included as a compound of the preparation, was synthesizedin 1950 in England, along the course of developing antimicrobial preparations for combating the causative agents of malaria. During that time, its high antibacterial activity, low toxicity, and pronounced ability to bind to the skin and mucous membranes were identified. Due to these properties, Chlorhexidine (CH) has come into wide use in the treatment of skin diseases, wounds, mucous membrane lesions, and in dentistry. In addition, CH is used as a preventive medication, a disinfectant agent for processing instruments, etc.

Chlorhexidine acts at the level of cell membrane, increasing its permeability. The first step in the action of Chlorhexidine is the rapid adsorption on the microbial wall, driven by the presence of two basic and symmetric groups of chlorophenylguanide attached to the hexamethylene lipophilic chain, which form a bicationic molecule interacting with the bacterial cell surface. The conditions for their binding are most favorable in a neutral or slightly alkaline pH; the amount of adsorbed Chlorhexidine depends on the concentration of the agent. The outer layer of the bacterial cell wall carries a negative charge, which usually stabilizes in the presence of cations such as Ca2+; this is the basis of action of most cationic antiseptics, including Chlorhexidine, having a high affinity for the bacterial cell wall.

Though the bisbiguanides' mechanism of action is very similar to the quaternary ammonium compounds, the former bind more prominently to phospholipids and proteins of the bacterial cell wall; the lengthy bisbiguanide hydrophobic part prevents their disconnection in the lipid bilayer. Thus, Chlorhexidine establishes a «bridge» between pairs of adjacent phospholipids, and displaces the corresponding divalent cations. The cationic Chlorhexidine molecules bind primarily to anionic compounds such as free sulfates, lipopolysaccharides, phosphate groups and protein carboxyl groups.

Low concentrations of Chlorhexidine reduce cytoplasmic membrane permeability, accompanied by the leakage of potassium ions, phosphate ions and protons, slowdown of respiratory processes and substance transport, changes in content, metabolism, and osmotic activity of enzymes. Changes in membrane integrity reflects the bacteriostatic effect of Chlorhexidine, and is reversible. Higher Chlorhexidine concentrations lead to the membrane crystallization, resulting in the loss of its structural integrity and catastrophic loss of intracellular matter. This is the basis of Chlorhexidine bactericidal action, which leads to precipitation or coagulation of the bacterial cytoplasm, with a paradoxical decrease in the components' outflow through the membrane. This process leads to eventual cell death. The effect development is dependent on the solution concentration and pH of the medium.

Chlorhexidine is an antiseptic having bactericidal and fungicidal effects. Although not considered a virucide, it has some observed activity against lipid membranes of viruses such as HIV, herpes 1 and 2, and influenza A. Studies of the relative Chlorhexidine activity against M53A 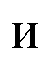 M 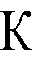 .8A (including those having plasmid resistance to gentamicin, propamidine isethionate, ethidium bromide) have revealed no differences in minimum inhibitory concentration (MIC) and killing effect.

Chlorhexidine is an antimicrobial preparation effective against gram-positive and gram-negative microorganisms, yeasts, and dermatophytes. It is active against Treponema pallidum, Chlamydia spp., Ureaplasma spp., Neisseria gonorrhoeae, Trichomonas vaginalis, Gardnerella vaginalis, Bacteroides fragilis, protozoa (Trichomonas vaginalis); viruses (herpes virus). Some strains of Pseudomonas spp. and Proteus spp. have low sensitivity to this preparation; also resistant are the acid-resistant forms of bacteria, bacterial spores, fungi, and viruses. Chlorhexidine does not interfere with the functional activity of the Lactobacilli. It remains active in the presence of blood and pus.

Methyl-4-hydroxybenzoate, ethyl-4-hydroxybenzoate and propyl-4- hydroxybenzoate constituents belong to the class of parabens. Each of the parabens has the activity spectrum with its own features. For example, methyl-4- hydroxybenzoate and propyl-4-hydroxybenzoate suppress more efficiently mould growth or yeast growth, respectively. Parabens are characterized by low toxicity, efficiency over a wide pH range, do not have a specific smell, color or taste, and donot cause sensory changes in products incorporating them. They are not mutagenic.

In addition, we have included ethyl-p-hydroxybenzoate in the novel preparation, which allows to further potentiate Chlorhexidine antimicrobial action. This agent exhibits the properties of a preservative and antiseptic, and exhibits some anesthetic effect. Thus, the preparation will obviate complications that may be caused by lidocaine. At the same time, the action of both Chlorhexidine itself, and the potentiating action of Chlorhexidine and the preservative, represented by formation of the additional antimicrobial complex, will be preserved; said complex, in turn, will be substantially reinforced by p-hydroxybenzoic acid ester, i.e. ethyl-4- hydroxybenzoate.

Furthermore, the preparation will have no irritant effect. Technical Effects:
1. No toxicity
2. Suitability for continuous application with no danger of developing resistance
3. No induction of a burning sensation (as with Hexicon in 20% of cases, orwith Instillagel in 10% of cases)
4. No interference with the functional activity of the Lactobacilli
5. No suppression (no changes) of the physiological pH
6. High efficiency against pathogens causing sexually transmitted diseases
7. Ability to destruct biofilms
8. Retaining activity in the presence of both physiological and pathological body fluids (blood, semen, pus)
9. Increase in activity upon pH shifts to the alkaline side
10. Creating conditions required for independent lactic acid bacterial flora recovery.

### EXAMPLES

Feasibility of the invention can be demonstrated by the following examples.

### Example 1 Gel composition

Chlorhexidine gluconate 0.05
gmethyl-4-hydroxybenzoate 0.06
ethyl-4-hydroxybenzoate 0.06
propyl-4-hydroxybenzoate 0.025
propylene glycol 5 g
hydroxyethyl cellulose 1.5
water up to 100 g

### Example 2 Gel composition

Chlorhexidine gluconate 0.5 g
methyl-4-hydroxybenzoate 0.03
ethyl-4-hydroxybenzoate 0.06
propyl-4-hydroxybenzoate 0.01
propylene glycol 70 g
hydroxyethyl cellulose 5 g
water up to 100 g

### Example 3

### A method for gel production

Chlorhexidine bigluconate, methyl-4-hydroxybenzoate, ethyl-4-hydroxybenzoate, propyl-4-hydroxybenzoate are added to a portion of purified water heated to 40°C, and stirred until completely dissolved; hydroxyethyl celluloseis then added, and stirred until homogeneity is achieved; propylene glycol is then added and stirred. The volume is adjusted to 100.0 using purified water. The process of mixing is continued until a homogeneous mass is obtained.

### Example 4 Study of the antimicrobial and antiseptic activity

Table 1 shows the minimum inhibitory concentrations (MIC) of Chlorhexidine in µg/ml, comprised in the claimed gel composition and prototype composition, against various microorganisms and fungi in the *in vitro* models.

**Table 1**

| Microorganism | Prototype | Claimed composition according to Example 1 |
|---|---|---|
| Pseudomonas aeruginosa | 80.0 | 76.0 |
| Staphylococcus aureus | 4.0 | 1.5 |
| Candida albicans | 4.0 | 2.4 |
| Porphyromonas gingivalis | 3.4 | 5.0 |
| Porphyromonas endodontalis | 3.4 | 6.0 |
| Prevotella melaninogenica | 3.4 | 3.0 |
| Prevotella intermedia | 3.4 | 3.1 |
| Enterococcus faecalis | 3.33 | 2.8 |
| Escherichia coli | 2.67 | 1.0 |
| Prevotella denticola | 2.67 | 3.0 |
| Streptococcus mutans | 1.0-2.0 | 0.8 |
| Enterobacter cloacae | ≤ 75 -≤150 | 50.0 |
| Klebsiella pneumoniae | ≤ 75-≤ 300 | 30.0 |
| Serratia marcescens | ≤150 | >100 |
| Pseudomonas maltophilia | ≤150 | 50.0 |
| Citoubacter diversus-levena | ≤37.5 | 29.0 |
| Enterobacter agglomerans | ≤75 | 52.0 |
| Klebsiella oxytoca | ≤300 | ≤150 |
| Streptococcus pneumonia | 5.0 | 3.4 |
| Staphylococcus aureus | 2.5 | 0.3 |
| Streptococcus oralis | 1.25 | 1.1 |
| Bacillus cereus | 0.04 | 0.63 |
| Klebsiella pneumonia | >10 | 5.8 |
| Serratia marcescens | 10.0 | 4.0 |
| Acanthamoeba polyphaga | 6.25 | 4.9 |
| Polyresistant Streptococcus mutans | 2.0-16.0 | 2.0 |
| Non-polyresistant Streptococcus mutans | 0.25-1.0 | 0.25 |
| Aspergillus spp. | 75-500 | 10.0 |
| Candida albicans | 7-15 | 5.0 |
| Microsporum spp. | 2.0-18.0 | 1.25 |
| Penicillium spp. | 150.0-200.0 | 50.0 |
| Saccharomyces spp. | 50.0-125.0 | 50.0 |
| Neisseria gonorrhoeae | 10.0 | 7.0 |
| Trichophyton spp. | 2.5-14.0 | 2.5 |
| Streptococcus sanguis | >100 | 30.0 |
| Actinomyces naeslundii | >100 | 125.0 |
| Bacillus subtilis | 10 | 10.0 |
| Proteus vulgaris | 20 | 20.0 |
| Serratia marcescens | 100 | 80.0 |

### Example 5 (Comparative example) Treatment of skin infections

In the sexual health clinic, a study on the efficacy and safety of the "Hexicon" preparation and the gel according to the invention was conducted in patients with acute and chronic dermatoses complicated by secondary infection. 60 patients were included in the study; a gel containing 0.05 g of Chlorhexidine was used topically.

Amongst dermatoses complicated by secondary infection, chronic eczema was diagnosed in 30% (n = 18) of patients, toxicodermatosis in 10% (n = 6), psoriasis in 10% (n = 6), contact dermatitis in 3.33% (n = 2), Darier's disease in 3.33% (n = 2), Ruiter's arteriolitis in 3.33% (n = 2). Acne vulgaris, in the papulopustular form, was revealed in 16.68% (n = 10) of patients, folliculitis in 10% (n = 63), cheilosis in 10% (n = 6), intertrigo in 3.33% (n = 2). The affected skin area varied from 0.5 to 18%. The duration of purulent disease ranged from a few days to3 months.

The results of the study showed that the clinical recovery occurred on Day 7 of the therapy in 20 persons, and on Day 14 in 10 persons treated with Hexicon; on Day 7 in 22 persons, on Day 10 in 2 persons, and on Day 14 in 6 persons treated with the gel of the invention. By the end of the treatment, the majority of patients inboth groups showed a decrease in subjective symptoms of the disease, such as itching, soreness, burning sensation; clinical signs of edema, erythema, excoriation, crusts, and pustules were almost completely resolved, while a mild skin lichenification, moderate skin dryness and peeling retained.

The bacteriological study in both groups of patients showed that the most common detected pathogenic microorganisms were the members of the gram- positive coccal flora, i.e. Staphylococcus aureus, Staphylococcus epidermidis. The results of the study revealed a positive Chlorhexidine effect on the skin bacterial load in patients with purulent infection; during treatment, both qualitative and quantitative changes in microbial flora composition were registered. Indeed, after the treatment, high counts (more than 10³ CFU/tampon) of Staphylococcus aureus emerged only in 4 and 2 of the respective patients; Staphylococcus epidermidis - in 1 patient (Hexicon), and Enterobacter cloacae - in 2 of 3 patients (Hexicon)

The researchers concluded that Chlorhexidine is efficient for treating mild to moderate dermatoses complicated by secondary infection.

### Example 6

Table 2 lists the comparative results of the antimicrobial action of the preparations

**Table 2**

| Preparatio nID | Staphylococcus aureus | E. coli | Pseudomonas aeruginosa | Candida |
|---|---|---|---|---|
| Instillagel | Bactericidal effect in 5 min | Bacteriostatic effect in 10 min, bactericidal effect in 15 min | Bactericidal effect in 5 min | Fungicida leffect in 5 min |
| According to Example 2 | Bactericidal effect in 5 min | Bactericidal effect in 5 min | Bactericidal effect in 3 min | Fungicida leffect in 5 min |

### Example 7 Study of the Chlorhexidine toxicity in acute and chronic experimentswith intravaginal gel administration.

The experiments were performed on (female) white rats and rabbits. In a chronic experiment, the influence of the intravaginally administered preparation on general well-being and behavior of the animals, the integral parameters characterizing basic metabolism, the cardiovascular system, liver and kidneys' functional state; morphological composition and biochemical parameters of peripheral blood, myelogram profile; and on pathomorphological and histological parameters, was studied in rats in doses of 4.6 and 46.0 mg/kg, and in rabbits in doses of 0.46 and 4.6 mg/kg (of the active principle). It was demonstrated that the intravaginal administration Chlorhexidine, in all doses tested, did not lead to development of pathological shifts in the functions of the organs and systems studied, does not cause dystrophic, destructive, focal sclerotic changes in parenchymal cells and stroma of the studied organs and has no local irritant effect upon intravaginal administration.

### Example 8 Study of preventive action against diseases caused by N.gonorrhoeae (NG) and/or C. trachomatis (CT)

312 nonpregnant females were enrolled in the study. The average age of the patients was 25.1 years. There were no statistically significant differences in place of residence, education, and familial status. 3 test groups included those not receiving antimicrobial treatment; those using post-coitally a commercially available prototype gel; and those using the gel according to Example 3 (on volunteers comprising 27% of the total number of subjects). The average number of sexual partners was 2.6 in all study groups. Laboratory samples were collected using a common procedure; NG was identified using cytochrome oxidase test and Gram staining. CT was isolated on McCoy cell culture, and stained with Lugol solution. NG and CT were isolated from 0.9% and 9.4% of the patients in the first group, 0.6% and 5.3% in the second group, and 0.32% and 2.88%, respectively.

Thus, the preparation can be efficiently used for individual prevention of sexually transmitted diseases after casual sexual encounters.

The preparation is further used as an antiseptic, as an antimicrobial preparation acting on gram-positive and gram-negative microorganisms, yeasts, and dermatophytes. It can be included in combination therapy of sexually transmitted infections, e.g. chlamidiosis, ureaplasmosis, gardnerellosis, trichomoniasis, gonorrhea, syphilis, and genital herpes. It maintains its activity in the presence of pus, blood and various secretions, and organic matter. The maximum antimicrobial activity is achieved in 5 minutes after application.

It is recommended to use the preparation immediately after unprotected sexual intercourse. The syrette contents are introduced into the male urinary tract by means of the nozzle or applicator designed for males, or to the female urethra and vagina by means of the nozzle or applicator designed for females, for 2-4 minutes. After the procedure, it is recommended to refrain from urination for 1 hour.

## Claims

1. Antiseptic pharmaceutical preparation in a gel form for use after casual sexual encounters in individual prevention of sexually transmitted diseases, wherein the preparation is for gel administration into a male or female urogenital tract, and wherein the preparation comprises the following components, in grams:
chlorhexidine gluconate 0.01-0.5
methyl 4-hydroxybenzoate 0.01-0.1
ethyl 4-hydroxybenzoate 0.01-0.1
propyl 4-hydroxybenzoate 0.01-0.1
propylene glycol 5-70
hydroxyethylcellulose 1.5-5
water up to 100.

2. The antiseptic pharmaceutical preparation for use according to claim 1, **characterized in that** it is prepackaged into disposable sterile syrettes with nozzles suitable for the gel administration into a male or female urogenital tract.

## Patentansprüche

1. Antiseptisches pharmazeutisches Präparat in Gelform zur Verwendung nach beiläufigen sexuellen Begegnungen bei der individuellen Vorbeugung von sexuell übertragbaren Krankheiten, wobei das Präparat zur Verabreichung des Gels in den männlichen oder weiblichen Urogenitaltrakt bestimmt ist, und wobei das Präparat die folgenden Inhaltsstoffe in Gramm umfasst:
Chlorhexidin-Gluconat 0,01-0,5
Methyl-4-hydroxybenzoat 0,01-0,1
Ethyl-4-hydroxybenzoat 0,01-0,1
Propyl-4-hydroxybenzoat 0,01-0,1
Propylenglykol 5-70
Hydroxyethylcellulose 1,5-5
Wasser bis zu 100.

2. Antiseptisches pharmazeutisches Präparat zur Verwendung gemäß Anspruch 1, **dadurch gekennzeichnet, dass** es in sterilen Einweg-Syretten mit Düsen vorverpackt ist, die für die Verabreichung des Gels in den männlichen oder weiblichen Urogenitaltrakt geeignet sind.

## Revendications

1. Préparation pharmaceutique antiseptique sous forme de gel pour un usage après des rapports sexuels occasionnels en prévention individuelle des maladies sexuellement transmissibles, selon laquelle la préparation est pour une administration du gel à l'intérieur d'un tractus urogénital masculin ou féminin, et selon laquelle la préparation comprend les composants suivants en grammes :
gluconate de chlorhexidine 0.01-0.5
4-hydroxybenzoate de méthyle 0.01-0.1
4-hydroxybenzoate d'éthyle 0.01-0.1
4-hydroxybenzoate de Propyle 0.01-0.1
propylène glycol 5-70
hydroxyéthylcellulose 1.5-5
eau jusqu'à 100.

2. Préparation pharmaceutique antiseptique pour un usage selon la revendication 1, **caractérisée en ce qu'**elle est préalablement conditionnée dans des syrettes stériles dotées de buses adaptées à l'administration du gel dans le tractus urogénital masculin ou féminin.
